# EUROPÄISCHE PATENTSCHRIFT

(11) **EP 0 471 890 B1**
(45) Veröffentlichungstag und Bekanntmachung des Hinweises auf die Patenterteilung: **19.10.1994**
(21) Anmeldenummer: 90124315.4
(22) Anmeldetag: 15.12.1990
(51) Int. Cl.: A23C 9/142, A23C 9/20, A61K 35/20, A61L 2/02

(54) **Sterilfiltrierte Kolostralmilch**
Sterile filtered colostrum
Colostrum stérilisé par filtration

(30) Priorität: 21.08.1990 DE 4026365
(43) Veröffentlichungstag der Anmeldung: 26.02.1992
(73) Patentinhaber: BIOTEST PHARMA GMBH, D-63303 Dreieich (DE)
(72) Erfinder: Hies, Henry, W-6074 Rödermark (DE); Möller, Wolfgang, Dr. Dipl.-Chem., W-6370 Oberursel (DE); Dichtelmüller, Herbert, Dr. Dipl.-Chem., W-6231 Sulzbach/Ts. (DE); Stephan, Wolfgang, Dr. Dipl.-Chem., W-6072 Dreieich (DE)
(74) Vertreter: Beil, Hans Chr., Dr.

(56) Entgegenhaltungen:
- EP-A- 46 909
- EP-A- 0 052 862
- EP-A- 0 173 999
- EP-A- 0 334 776
- EP-A- 0 363 896
- WO-A-86/01687
- DE-A- 3 743 440
- US-A- 4 140 806

## Beschreibung

Die Erfindung betrifft die in den Ansprüchen näher gekennzeichnete Kolostral-Milch und ein Verfahren zu ihrer Herstellung.

Kolostral-Milch vom Rind, die in den ersten 30 Stunden nach dem Kalben entnommen wurde, enthält eine hohe Konzentration an IgG, IgA und IgM und ist auch in ihrer sonstigen Proteinzusammensetzung optimal dafür geeignet, den Kälbern in den ersten Tagen nach der Geburt eine passive Immunität speziell gegen enteropathogene Keime zu verleihen. Diese Eigenschaft der Kolostral-Milch hat dazu geführt, daß mehrere Verfahren entwickelt wurden, die Kolostralmilch-Immunglobuline zu isolieren und für eine Therapie speziell von gastrointestinalen Störungen beim Menschen zu verwenden.

So beschreiben z.B. DE-C-2 813 984, DE-C-3 432 718, EP-B-102 831 und US-A-4 051 235, US-A-4 784 850 und US-A-4 834 974 die Herstellung von immunglobulinhaltigen Präparaten aus KolostralMilch. Diese Präparate enthalten neben den Immunglobulinen teilweise noch andere Proteine. Allen Präparaten gemeinsam ist aber, daß sie aus Kolostral-Molke hergestellt worden sind, nachdem zuvor das Casein durch Säurefällung oder enzymatische Prozesse präzipitiert und abgetrennt worden ist. Die Abtrennung des Caseins ist in all den Fällen notwendig, in denen die Kolostral-Milch entweder sterilfiltriert oder ultrafiltriert werden soll.

Kolostral-Milch vom Rind hat eine Proteinkonzentration von rund 12 % und enthält neben zellulären Partikeln eine hohe Zahl an Bakterien.

Der Keimgehalt in der rohen Kolostral-Milch liegt bei 10⁶ - 10⁸ Keimen pro ml, im Vergleich zu 10³ - 10⁴ Keimen pro ml bei normaler Kuhmilch. Durch Pasteurisation z.B. für 15 bis 40 sec bei 71 - 74 °C oder für 1 bis 4 sec bei 85 - 90 °C läßt sich zwar der Gehalt an pathogenen Keimen um 1 - 2 Zehnerpotenzen reduzieren, die Milch bleibt aber immer noch sehr stark mit Bakterien belastet. Bei der Lagerung können sich diese oder neu eingetragene Keime wieder vermehren und besonders bei der therapeutischen Anwendung der Kolostral-Milch bei gastrointestinalen Störungen zu ernsten Nebenwirkungen führen.

In US-A-3 911 108 ist die Sterilisation von Kolostral-Milch und ihrer Derivate mit β-PL beschrieben. Eine andere Methode stellt z.B. die gamma-Bestrahlung der Kolostral-Milch dar. Beide Methoden führen aber zu einer teilweisen Denaturierung der Proteine.

US-A-4 140 806 beschreibt das Filtrieren von normaler Milch zum Abtrennen des Fettes und gleichzeitigem Sterilmachen des Filtrats. Diese Milch aber hat eine grundsätzlich andere Protein- und Fettzusammensetzung als Kolostral-Milch, die auch eine wesentlich höhere Keimbelastung aufweist.

GB-A-2 052 979 lehrt die Sterilisation entfetteter Kolostral-Milch durch ionisierende Strahlung. Während durch eine Sterilfiltration die pathogenen Bakterien komplett abgetrennt werden, führt die durch Bestrahlung bewirkte Abtötung von Bakterien dazu, daß die Milch dann vermehrt Toxine und Zellbestandteile der Bakterien enthält.

EP-A-0 046 909 beschreibt eine Kolostral-Milch von während der Trächtigkeit immunisierten Kühen, bei der durch Zentrifugieren Bakterien und Fett abgetrennt werden. Eine völlig keimfreie Milch ist durch Zentrifugieren aber nicht erhältlich.

Die einfachste, effektivste und proteinschonendste Methode zur Entfernung der Bakterien, die Sterilfiltration, ist mit Kolostral-Milch nicht möglich, weil insbesondere das Casein sofort den Filter verstopft. Aus diesem Grunde verwenden alle Verfahren, die zu einem sterilfiltrierbaren oder ultrafiltrierbarem Präparat gelangen wollen, die gegebenenfalls entfettete Molke als Ausgangsmaterial. Bei der Herstellung der Molke wird das Casein durch Säure- oder Enzymwirkung ausgefällt und durch Zentrifugation abgetrennt. Die resultierende Molke bzw. die aus ihr isolierten Fraktionen sind dann ultrafiltrierbar und sterilfiltrierbar.

In DE-C-2 813 984 wird ausdrücklich darauf hingewiesen, daß die Entrahmung und Klärung (Caseinfällung) sehr weit getrieben werden muß, um eine nachfolgende Verstopfung der Filter und Ultrafilter zu vermeiden.

Die Entfernung des Caseins durch Präzipitation hat aber gravierende Nachteile für die weitere Verwendung bzw. Aufreinigung der Kolostral-Milch:
1. Durch Einschluß in den Niederschlag und Copräzipitation mit dem Casein kann der Verlust an Immunglobulinen bei diesem Schritt bis zu 40 % betragen.
2. Die Zentrifugation des Casein-Niederschlags ist ein aufwendiger Schritt, der um so aufwendiger wird, je mehr man auf die Ausbeute der Immunglobuline achtet.
3. Casein selbst hat günstige therapeutisch anwendbare Eigenschaften, die besonders die Wirkung der Immunglobuline bei gastrointestinalen Störungen unterstützen. So werden aus dem Casein opiatartige Wirkstoffe freigesetzt, die zur Hemmung der Darmbewegung und Förderung der Elektrolyt- und Wasserresorption führen. Diese Wirkungen werden auch dem intakten Casein zugeschrieben (Recker, B., Physis 1/89, 32).

Generell wäre es wünschenswert, die Kolostral-Milch möglichst wenig in ihrer Proteinzusammensetzung zu verändern, da sie geradezu optimal für die Prophylaxe und Therapie von gastrointestinalen Infektionen und Störungen zusammengesetzt ist. So haben neben den Immunglobulinen und dem Casein andere Proteine wie Lysozym, Lactoferrin und Peroxydasen wichtige Funktionen bei der Abwehr bakterieller Infektionen. Ein Großteil dieser wichtigen Proteine werden bei der Casein-Fällung und der Pasteurisation aber abgetrennt oder inaktiviert.

Die Erfindung hatte deshalb zum Ziel, ein Verfahren zur Entfernung der Bakterien aus Kolostral-Milch durch Sterilfiltration zu entwickeln, bei dem möglichst alle Proteinbestandteile der ursprünglichen Kolostral-Milch weitgehend erhalten bleiben.

Es wurde nun überraschenderweise gefunden, daß man Kolostral-Milch klärfiltrieren und sterilfiltrieren kann, ohne vorher die Casein-Fraktion zu entfernen, wenn man die gegebenenfalls entfettete Kolostral-Milch auf einen pH-Wert von unter 3,5 ansäuert. Beim Herabsenken des pH-Werts fällt zwar das Casein bei pH 4 bis 5 aus, wird aber durch das weitere Absenken des pH-Werts wieder in Lösung gebracht. Diese saure Lösung ist in ihren Eigenschaften gegenüber der Kolostral-Milch soweit verändert, daß sie nun sterilfiltrierbar ist.

Man kann die auf diese Weise angesäuerte Kolostral-Milch auch vor der Filtration wieder auf ihren Ausgangs-pH-Wert einstellen. Bei diesem Zurückstellen des pH-Werts fällt das Casein bei pH 4 bis 5 erneut aus und geht ab etwa pH 5,5 wieder in Lösung. Die so erhaltene Kolostral-Magerilch ist überraschenderweise sogar noch etwas besser filtrierbar als die saure Kolostral-Magermilch mit einem pH-Wert von unter 3,5.

Beim Herabsenken des pH-Wertes ist es wichtig, daß das Casein nur kurz in dem präzipierten Zustand verbleibt und der pH-Wert zügig weiter gesenkt wird, bis das Casein wieder in Lösung gebracht worden ist. Wenn der Casein-Niederschlag zu lange als Präzipitat vorliegt und erst nach längerer Zeit wieder in Lösung gebracht worden wird, wird die Filtrierbarkeit der Kolostral-Milch deutlich schlechter.

Eine bevorzugte Ausführungsform der Erfindung besteht darin, daß man Kolostral-Milch durch an sich bekannte Maßnahmen entfettet und mit NaCl-Lösung auf einen Protein-Gehalt von 20 g/l verdünnt. Durch schnelle Zugabe von Salzsäure wird der pH-Wert der Kolostral-Magermilch auf pH 2,0 bis 3,5 vorzugsweise 2,8 bis 3,2 eingestellt, bis der bei der Ansäuerung entstandene Niederschlag wieder gelöst ist. Die Temperatur sollte bei der Säurebehandlung nicht über 45 °C liegen, um eine Denaturierung der Immunglobuline zu vermeiden.

Gegebenenfalls wird der pH-Wert sofort oder nach einiger Zeit wieder mit Natronlauge auf den ursprünglichen Wert der Kolostral-Magermilch eingestellt. Bei diesem Hochstellen des pH-Werts fällt das Casein zeitweilig wieder aus und geht sofort wieder in Lösung.

Die dieser Behandlung unterzogene Kolostral-Magermilch kann nun mit den bekannten Methoden leicht sterilfiltriert oder ultrafiltriert werden. Zum Beispiel kann man die Kolostral-Magermilch über ein Tiefenfilter klärfiltrieren. Die auf solche Weise klärfiltrierte Kolostral-Magermilch kann dann über Membran- oder Tiefenfilter sterilfiltriert werden. Auch eine Ultrafiltration oder Diafiltration der erfindungsgemäß behandelten Kolostral-Magermilch vor oder nach der Sterilfiltration ist möglich.

Die Filtrierbarkeit läßt sich weiter verbessern, wenn man Filterhilfsmittel wie z.B. Hyflo Supercell Ⓡ bei der Filtration verwendet.

Die sterilfiltrierte Kolostral-Magermilch wird vorzugsweise auf einen Proteingehalt von 20 bis 70 g/l eingestellt, kann aber auch bis auf 120 g/l konzentriert werden. Durch Diafiltration kann die Konzentration der Laktose reduziert und das Ionenmilieu derart eingestellt werden, daß das erhaltene Präparat besonders für die therapeutische Anwendung bei gastrointestinalen Störungen geeignet ist. Die sterilfiltrierte Kolostral-Magermilch kann außerdem gefriergetrocknet oder in ihre Proteinbestandteile fraktioniert werden.

Wenngleich die Filtration vorzugsweise mit der entfetteten Kolostral-Magermilch durchgeführt wird, eignet sich das erfindungsgemäße Verfahren auch für nicht entfettete Kolostral-Milch. Die Filtrationsrate ist zwar deutlich geringer als bei der Kolostral-Magermilch, aber immer noch wesentlich besser als bei der nicht säurebehandelten Kolostral-Milch.

Die folgenden Beispiele sollen die Erfindung näher beschreiben.

### Beispiel 1:

500 ml gefrorene Kolostral-Milch wurden mit 500 ml Wasser versetzt und bei 37°C aufgetaut. Durch Zentrifugation wurde die Lipid-Fraktion abgetrennt und die so erhaltene Kolostral-Magermilch mit 2000 ml 100 mM NaCl-Lösung verdünnt.

Mit 1 N Salzsäure wurde der pH-Wert innerhalb von 30 Sekunden auf pH 3,0 eingestellt. Nach einer Stunde wurde die Lösung (ca. 3 l) mit einem Druck von 0,5 bar über einen Tiefenfilter mit 150 cm² Filterfläche (Supra 80 der Fa. Seitz) klärfiltriert. Im Anschluß an die Klärfiltration wurde über eine Membranfilter-Kombination mit 5µ/1,2µ/0,65µ/0,22µ Membranen sterilfiltriert.

Zum Vergleich wurde die Kolostral-Milch in gleicher Weise entfettet und verdünnt, allerdings ohne das Ansäuern auf pH 3,0. Von dieser Kolostral-Magermilch konnten über die Tiefenfilter nur 150 ml unter hohem Druck bei 3 bar filtriert werden. Eine Sterilfiltration über Membranfilter war nicht möglich.

### Beispiel 2:

500 ml Kolostral-Milch wurden wie im Beispiel 1 auf pH 3,0 angesäuert. Nach zwei Stunden wurde der pH-Wert auf 6,5 eingestellt und in gleicher Weise wie in Beispiel 1 klär- und sterilfiltriert. Diesmal war die Filtration über den Tiefenfilter bei einem Druck von 0,2 bar möglich.

### Beispiel 3:

500 ml Kolostral-Milch wurden wie in Beispiel 2 behandelt und filtriert. Bei der Tiefenfiltration über Supra 80-Filter wurden 3 g Filterhilfsmittel (Hyflo Supercell) pro 100 ml verdünnte Kolostral-Magermilch zugesetzt. Dieser Zusatz verbesserte die Filtrationsrate bei der Tiefenfiltration. Anschließend wurde wie in Beispiel 1 sterilfiltriert.

Eine Kontroll-Präparation Kolostral-Magermilch ohne Ansäuern auf pH 3,0 wie in Beisiel 1 konnte nach Zusatz des Filterhilfsmittels bei einem Druck von 1 bar über den Supra 80 Tiefenfilter klärfiltriert werden. Die Sterilfiltration über Membranfilter war aber nicht möglich.

### Beispiel 4:

500 ml Kolostral-Milch wurden wie im Beispiel 1 entfettet und anschließend unverdünnt auf pH 2,8 eingestellt. Anschließend wurde der pH-Wert wieder auf 6,5 eingestellt. Die Kolostral-Magermilch wurde über Supra 80 Tiefenfilter unter Zusatz von 3 % Filterhilfsmittel klärfiltriert und anschließend über EK 1-Filter (Fa. Seitz) sterilfiltriert.

### Beispiel 5:

500 ml Kolostral-Milch wurden mit 2500 ml 80 mM NaCl-Lösung verdünnt und mit 1 N Salzsäure auf pH 3,1 eingestellt. Der pH-Wert wurde anschließend auf pH 7,0 eingestellt und die fetthaltige Kolostral-Milch über Supra 80 Tiefenfilter unter Zusatz von 3 % Filterhilfsmittel (Hyflo Supercell) klärfiltriert und anschließend über EK 1-Sterilfilter sterilfiltriert.

Obwohl die Filtrationsrate bei der Klär- und bei der Sterilfiltration schlechter war als mit der entfetteten Kolostral-Magermilch, ist doch eine Filtration mit entsprechend größerer Filterfläche möglich. Die nicht angesäuerte, nicht entfettete Kolostral-Milch war unter den gleichen Bedingungen nicht einmal über den Supra 80-Tiefenfilter zu filtrieren.

### Beispiel 6:

1 Liter der sterilfiltrierten Kolostral-Magermilch aus Beispiel 3 wurde über eine 10000 D-Membran mit einer Membranfläche von 0,9 m² gegen das 5-fache Volumen 80 mM NaCl-Lösung diafiltriert und anschließend über die gleiche Membran ultrafiltriert.

Zum Vergleich wurde eine in gleicher Weise verdünnte, aber nicht angesäuerte Kolostral-Magermilch der gleichen Diafiltration und Ultrafiltration unterzogen.

Bei der gewählten Versuchsanordnung betrug die Permeat-Flußrate für die erfindungsgemäß behandelte Kolostral-Magermilch 11 l pro Stunde und m² Membranfläche bei einem Transmembrandruck von 1,2 bar. Bei der Ultrafiltration wurde die Kolostral-Magermilch auf einen Proteingehalt von 110 g pro l ankonzentriert.

Bei der Vergleichs-Präparation stieg schon während der Diafiltration der Transmembrandruck innerhalb kurzer Zeit auf über 2 bar an, und die Flußrate nahm rapide ab. Wegen Verstopfung der Membran mußte die Diafiltration abgebrochen werden. Eine Ultrafiltration war erst gar nicht möglich.

## Patentansprüche

1. Sterilfiltrierte Kolostral-Milch, dadurch gekennzeichnet, dass
- der natürliche Caseingehalt nicht oder nur unwesentlich reduziert ist
- alle Bakterien durch Sterilfiltration abgetrennt worden sind
- sie in einem ersten Schritt auf einen pH-Wert von unter 3,5 angesäuert wurde, um das zunächst ausfallende Casein wieder in Lösung zu bringen und anschliessend die erhaltene Lösung sterilfiltriert wurde.

2. Milch nach Anspruch 1, dadurch gekennzeichnet, dass die Kolostral-Milch vom Rind stammt.

3. Milch nach Anspruch 1 oder 2, dadurch gekennzeichnet, dass die Kolostral-Milch entfettet ist.

4. Milch nach einem der Ansprüche 1 bis 3, dadurch gekennzeichnet, dass der Proteingehalt 1 bis 120 g pro l beträgt.

5. Milch nach Anspruch 4, dadurch gekennzeichnet, dass der Proteingehalt 1 bis 50 g pro l beträgt.

6. Milch nach einem der Ansprüche 1 bis 5, dadurch gekennzeichnet, dass der Laktosegehalt unter 100 mg pro l beträgt.

7. Verfahren zur Gewinnung einer sterilfiltrierten, Caseinhaltigen Kolostral-Milch nach einem der Ansprüche 1 bis 6, dadurch gekennzeichnet, dass man
a) die gegebenenfalls verdünnte Kolostral-Milch soweit ansäuert, dass das zunächst ausfallende Casein wieder in Lösung gegangen ist und
b) die erhaltene Lösung sterilfiltriert.

8. Verfahren nach Anspruch 7, dadurch gekennzeichnet, dass man den pH-Wert der Kolostral-Milch auf 2,5 - 3,5 einstellt.

9. Verfahren nach Anspruch 7 oder 8, dadurch gekennzeichnet, dass man den pH-Wert vor der Filtration wieder auf pH 5,5 - 8 einstellt.

10. Verfahren nach einem der Ansprüche 7 bis 9, dadurch gekennzeichnet, dass man vor der Sterilfiltration eine Klärfiltration durchführt.

11. Verfahren nach einem der Ansprüche 7 bis 10, dadurch gekennzeichnet, dass man bei der Filtration Filterhilfsmittel zusetzt.

12. Verfahren nach einem der Ansprüche 7 bis 11, dadurch gekennzeichnet, dass man die Kolostral-Milch vor der Säurefällung entfettet.

13. Verfahren nach einem der Ansprüche 7 bis 12, dadurch gekennzeichnet, dass die Klär- und die Sterilfiltration über Tiefen- oder Membranfilter erfolgt.

## Claims

1. Sterile-filtered colostrum, characterized in that
- the natural casein content is not reduced or is only insignificantly reduced,
- all bacteria have been separated out by sterile filtration, and
- it has been acidified in a first step to a pH of less than 3.5 in order to redissolve the initially precipitated casein, and the resulting solution has then been sterile-filtered.

2. Sterile-filtered colostrum according to Claim 1, characterized in that the colostrum originates from cows.

3. Sterile-filtered colostrum according to Claim 1 or 2, characterized in that the colostrum has had the fat removed.

4. Sterile-filtered colostrum according to one of Claims 1 to 3, characterized in that the protein content is 1 to 120 g per l.

5. Sterile-filtered colostrum according to Claim 4, characterized in that the protein content is 1 to 50 g per l.

6. Sterile-filtered colostrum according to one of Claims 1 to 5, characterized in that the lactose content is less than 100 mg per l.

7. A method of preparing a sterile-filtered, casein-containing colostrum according to one of Claims 1 to 6, characterized in that
a) the optionally diluted colostrum is acidified until the initially precipitated casein has redissolved, and
b) the resulting solution is sterile-filtered.

8. A method according to Claim 7, characterized in that the pH of the colostrum is adjusted to 2.5 - 3.5.

9. A method according to Claim 7 or 8, characterized in that the pH is readjusted to 5.5 - 8 before filtration.

10. A method according to one of Claims 7 to 9, characterized in that a clarification is carried out before the sterile filtration.

11. A method according to one of Claims 7 to 10, characterized in that filter aids are added in the filtration.

12. A method according to one of Claims 7 to 11, characterized in that the fat is removed from the colostrum before the acid precipitation.

13. A method according to one of Claims 7 to 12, characterized in that the clarification and sterile filtration are carried out using depth-type or membrane filters.

## Revendications

1. Lait colostral filtré en milieu stérile, caractérisé en ce que :
- la teneur en caséine naturelle n'est pas diminuée ou que de façon insensible
- toutes les bactéries ont été séparées par filtration stérile
- le lait a été acidifié au cours d'une première étape à un pH inférieur à 3,5, pour redissoudre la caséine tout d'abord précipitée et
- la solution obtenue a été ensuite soumise à une filtration stérile.

2. Lait selon la revendication 1, caractérisé en ce que le lait colostral provient de bovin.

3. Lait selon la revendication 1 ou 2, caractérisé en ce que le lait colostral est dégraissé.

4. Lait selon une des revendications 1 à 3, caractérisé en ce que la teneur en protéines est comprise entre 1 et 120 g/l.

5. Lait selon la revendication 4, caractérisé en ce que la teneur en protéines est comprise entre 1 et 50 g/l.

6. Lait selon une des revendications 1 à 5, caractérisé en ce que la teneur en lactose est inférieure à 100 mg/l.

7. Procédé d'obtention d'un lait colostral contenant de la caséine, soumis à une filtration stérile, selon une des revendications 1 à 6, caractérisé en ce que
a) on acidifie le lait colostral éventuellement dilué jusqu'à ce que la caséine tout d'abord précipitée se redissolve et
b) on soumet la solution obtenue à une filtration stérile.

8. Procédé selon la revendication 7, caractérisé en ce qu'on ajuste le pH du lait colostral à 2,5-3,5.

9. Procédé selon la revendication 7 ou 8, caractérisé en ce qu'on réajuste le pH à 5,5-8 avant la filtration.

10. Procédé selon une des revendications 7 à 9, caractérisé en ce qu'on procède à une filtration clarifiante avant la filtration stérile.

11. Procédé selon une des revendications 7 à 10, caractérisé en ce qu'on ajoute des auxiliaires de filtration au cours de la filtration.

12. Procédé selon une des revendications 7 à 11, caractérisé en ce qu'on dégraisse le lait colostral avant la précipitation à l'acide.

13. Procédé selon une des revendications 7 à 12, caractérisé en ce que la filtration clarifiante et la filtration stérile s'effectuent sur des filtres à lit profond ou à membrane.
